Europäisches Patentamt

European Patent Office

Office européen des brevets

(19)

(11) Publication number: **0 210 863**

**A1**

(12) # EUROPEAN PATENT APPLICATION

(21) Application number: 86305828.5

(22) Date of filing: 29.07.86

(51) Int. Cl.4: **G01N 33/52** , G01N 33/535 , G01N 33/536 , G01N 33/577 , C07C 87/50

A request for correction on pages 3 and 4 of the description has been filed pursuant to Rule 88 EPC. A decision on the request will be taken during the proceedings before the Examining Division (Guidelines for Examination in the EPO, A-V, 2.2).

(30) Priority: 29.07.85 US 760114

(43) Date of publication of application: 04.02.87 Bulletin 87/06

(84) Designated Contracting States: AT BE CH DE FR GB IT LI LU NL SE

(71) Applicant: **MODERN DIAGNOSTICS, INC.** **5895 Power Inn Road** **Sacramento California 95824(US)**

(72) Inventor: **Buck, Robert L.** **3940 G. Parkway** **Sacramento California 95823(US)** Inventor: **Gibson, Rene G.** **8725 LaRiviera Drive No. 7** **Sacramento California 95826(US)**

(74) Representative: **Sheard, Andrew Gregory et al** **Kilburn & Strode 30, John Street** **London WC1N 2DD(GB)**

(54) Immunoassay.

(57) An improved immunoassay featuring, in one aspect, the use of a liquid tetramethylbenzidine composition at a pH between 7.0 and 9.5.

EP 0 210 863 A1

## IMMUNOASSAY

This invention relates to the detection of antigens and antibodies in biological samples from human patients, e.g., serum or urine. (Herein, an antigen and an antibody to that antigen are referred to as a "specific binding pair.")

A great many antigens and antibodies are currently detectable by immunoassay methods in which the first member of the specific binding pair is detected by its reaction with the second member to form immune complexes. Some such assays employ a conjugate of the second member of the pair with an enzyme (e.g., horseradish peroxidase, or "HRP") which typically acts on a substrate (e.g., peroxide) to cause a colour change in a colour reagent (e.g., tetramethylbenzidine, or "TMB", in an acidic medium). Such conjugates generally are stabilized, prior to use, in buffers containing sulphate and/or phosphate.

One commonly detected antigen is human chorionic gonadotropin ("hCG"), a hormone which becomes present in the blood and urine in appreciable amounts during pregnancy. Since the alpha chain of hCG is the same as that of several other fertility hormones, it is the beta chain which is commonly detected, using anti-beta hCG antibodies. Such pregnancy tests are described in many U.S patents, e.g., Schurrs et al. U.S. Pat. No. 3,654,090, hereby incorporated by reference.

In general, the invention features improvements in the above-described type of immunoassays.

One such improvement is the use of TMB at an alkaline pH, i.e., between 7.0 and 9.5, which results in increased colour reagent stability, superior colour development, and the ability to stain solid substrates (e.g., polystyrene reaction tubes) to provide a permanent record of a positive reaction.

Another such improvement is the conjugation of one member of the specific binding pair with an enzyme such as HRP using a g-MBS-SATA linkage (described in greater detail below); this linkage provides enhanced enzyme stability and specificity of reactants.

Another such improvement is the use of coated supports (generally, tubes) in which the coating is a mixture of polyclonal and monoclonal antibodies; preferably, there are three or more different monoclonal antibodies, and the protein polyclonal:monoclonal ratio is 5:1 to 8:1, most preferably about 7:1.

The improvements of the invention permit the screening of human serum or urine for the presence of $\beta$-hCG in systems featuring speed, simplicity, sensitivity, and accuracy. Because the detection system is stable over long periods of time, it can be sold over the counter as a pregnancy detection kit for home use, or it can be used in a clinical laboratory or doctor's office.

Other features and advantages of the invention will be apparent from the following description of the preferred embodiments thereof, and from the claims.

Description of the Preferred Embodiments

A $\beta$-hCG test of the invention is described below.

Apparatus

A $\beta$-hCG test apparatus contains four components:

1. polystyrene tubes coated on the inner surface with a mixture of polyclonal anti-$\beta$-hCG antibodies and monoclonal anti-$\beta$-hCG antibodies;

2. monoclonal anti-$\beta$-hCG antibody conjugated with horseradish peroxidase (HRP) via an N-omega-maleimidobutyrloxy-succinimide (g-MBS)-N-succinimidyl-S-acetyl-thioacetate (SATA) linkage;

3. tetramethylbenzidine (TMB) indicator dye in an alkaline buffered solution maintained at approximately pH 8.0; and

4. a peroxide substrate.

The $\beta$-hCG test apparatus can be provided in two forms. In one form, suitable for use in a clinical laboratory or doctor's surgery, the four components are provided separately. In another form, suitable for use in the home, the antibody-HRP conjugate in lyophilized form is sealed in the coated polystyrene tube and the peroxide substrate is provided in the form of a tablet affixed to the cap of the tube, or is packaged separately.

A detailed description of the preparation of the individual components follows.

Antibody-coated Tubes

To coat the inner surface of a clean polystyrene tube (12 x 75mm), a 7:1 protein:protein mixture of polyclonal rabbit anti-$\beta$-hCG antibodies against $\beta$-hCG and monoclonal anti-$\beta$-hCG antibodies is diluted in 0.10M NaHCO₃ buffer (pH 8.1) to a final dilution of 1:500 (20 g/ml). The monoclonal antibodies represent a mixture of 5 different monoclonal antibodies obtained using standard hybridoma technology. One ml of the diluted antibody solution is placed in the tube using a glass pipette, after which the tube is incubated at 20°C for 18-24 hours. The antibody solution is then aspirated from the tube, and the tube is washed with 1.0 ml of 0.15 $\underline{M}$ NaCl in deionized water. Four ml of a solution of 1% BSA in Tris buffer (pH 8.0) is then added to the tube and the tube is incubated at room temperature for 18-24 hours, after which the BSA solution is aspirated from the tube and the tube is dried at 37°C for 18-24 hours. The coated tube is then heat sealed with dessication and stored until further use.

HRP-Anti-$\beta$-hCG Antibody Conjugate

The HRP-antibody conjugate is prepared by synthesizing the g-MBS derivative of anti-$\beta$-hCG monoclonal antibody and the deacetylated SATA derivative of HRP; the two derivatives are then reacted to form the conjugate containing three HRP molecules/antibody molecule. The g-MBS derivative of anti-$\beta$-hCG monoclonal antibody is prepared as follows.

Ten mg of anti-$\beta$-hCG monoclonal antibody - (mol. wt. 150,000) prepared using standard hybridoma technology is dialyzed against 0.1 $\underline{M}$ phosphate (pH 7.1) overnight at 4°C with constant stirring. After dialysis, the antibody solution is filtered and the antibody concentration adjusted to 5.0 mg/ml. 37.5. l of a stock solution of g-MBS in DMSO (10mg/ml) is then added dropwise to 2ml of the antibody solution with stirring. After 5 minutes, another 37.5 l of g-MBS solution is added (final antibody/g-MBS molar ratio = 1:40) and the entire mixture is stirred for 5 minutes following the addition. The mixture is then passed over an equilibrated G-25 desalting column, followed by dialysis to remove free g-MBS completely. The g-MBS-derivatized antibody is then added to the SATA derivative of HRP, which is prepared as follows.

SATA (6.93 mg) is dissolved in 200 ml DMSO and 119 l of the solution is added with stirring to a solution of 50 mg HRP in 0.05 $\underline{M}$ phosphate EDTA buffer (4.0mg/ml, pH 7.5). The resulting mixture is allowed to react for 10 minutes to yield 1 mol sulphydryl groups/mol HRP. The reaction mixture is then desalted on a Sephadex G-25 desalting column previously equilibrated with phosphate EDTA buffer and the brown peak is collected for deacetylation.

To deacetylate the SATA-HRP derivative, 100 ml of a 50 m$\underline{M}$ aqueous solution of hydroxylamine containing 2.5 m$\underline{M}$ EDTA is added to the SATA-HRP derivative and the mixture is stirred at room temperature for 1 hour. The mixture is then desalted on a Sephadex G-25 desalting column previously equilibrated with phosphate EDTA buffer and the brown peak, containing the deacetylated SATA-HRP derivative, is collected.

Fourteen mg of the deacetylated SATA-HRP derivative is combined with 16.5mg of the g-MBS-antibody derivative (antibody-enzyme molar ratio = 1:3) and incubated for 1 hour at room temperature while stirring to yield the HRP-antibody conjugate. The conjugate is then dialyzed overnight in a stabilized buffer solution containing 50 mM Tris, pH 7.2; 10 mM EDTA/Iron (III); 10% D-mannitol; and 2% sucrose. The dialyzed conjugate is then stored for future use in the stabilized buffer solution or in lyophilized form.

Alkaline Buffered TMB Solution

Sixty mg TMB (hydrochloride form), 120mg dioctylsulphosuccinate, and 20ml 70% isopropyl alcohol are combined with stirring at room temperature. Four ml of this solution is then added dropwise to 100ml of 0.10 $\underline{M}$ Tris (pH 8.1) at room temperature with stirring, taking care to maintain the pH of the solution at approximately 8.0. The solution is then filtered through a 0.2 micron Nalgene sterile filter and stored at room temperature in an amber bottle or opaque plastic vial until needed.

Peroxide Substrate

The peroxide substrate may be in the form of a solution e.g., 0.3% $H_2O_2$ or 0.25% urea peroxide in citrate buffer, or a tablet which readily dissolves, e.g., a tablet containing urea peroxide, NaCl, sugar, and excipients to hold the tablet together.

Use

The $\beta$-hCG test apparatus described above can be used to detect pregnancy in a female patient as follows.

A urine or serum specimen from the patient is dispensed into the antibody-coated tube and an equal volume of the antibody-HRP conjugate in stabilization buffer is added. Alternatively, the conjugate is already present in the tube in lyophilized form and is dissolved by the specimen.

The mixture is allowed to incubate for 3-5 minutes, and the tube is then rinsed thoroughly with a wash solution and/or water. One ml of the TMB buffer solution is then added to the tube, followed by the peroxide substrate. If the peroxide substrate is in the form of a tablet affixed to the cap of the tube, the capped tube is shaken to dissolve the peroxide tablet.

If the specimen contains β-hCG, both the coated test tube surface and the liquid will turn blue, giving a positive pregnancy test. A negative will not coat the coated test tube surface.

## Mechanism

When a serum or urine sample containing β-hCG antigen is incubated in the antibody-coated tube with the antibody-HRP conjugate, the antigen binds to the tube surface at one set of binding sites and to the conjugate at a separate binding site, forming an HRP-labelled antibody sandwich. Non-binding sample constituents are washed away.

The HRP label acts on the peroxide substrate to oxidize TMB to produce a blue colour both in solution and on the walls of the tube. By colouring the walls of the tube, the test minimizes the risk of false positives due to incomplete washings. In addition, the tube provides a permanent record of the test results because the colour remains stable on the coated surface of the tube indefinitely. It is believed that maintaining the TMB solution at approximately pH 8.0, rather than at acidic pH, is at least in part responsible for colouring both the solution and the walls of the tube.

## Other Embodiments

Other embodiments are within the following claims.

For example, the improvements of the invention are applicable to any immunoassays, e.g., for other hormones, drugs, or toxins, in any of the wide variety of colourometric immunoassay formats, including microliter plates. Any of the other components can also be varied, as is conventional, e.g., other tetraionic chelating agents, e.g., ethylene glycol bis (beta amino-ethyl ether) N,N tetraacetic acid can be used in place of EDTA in the stabilization buffer.

## Claims

1. A liquid composition of tetramethylbenzidine, buffered to a pH between 7.0 and 9.5.

2. The composition of Claim 1, said composition being an aqueous solution.

3. The composition of Claim 1 or Claim 2, buffered to a pH of about 8.0.

4. An immunoassay method in which a first member of a specific binding pair is detected by the reaction of said first member of said specific binding pair with the second member of said pair to form immune complexes, and the detection of such immune complexes by the addition to said reaction vessel of a liquid composition of tetramethylbenzidine, characterized in that said liquid composition of tetramethylbenzidine is buffered to a pH between 7.0 and 9.5.

5. A solid support having adhered thereto a coating comprising antibody to a predetermined antigen, characterized in that said coating comprises a polyclonal antibody and at least one monoclonal antibody.

6. A support as claimed in Claim 5, wherein said coating comprises at least three different monoclonal antibodies against said antigen.

7. A support as claimed in Claim 6, wherein said coating contains a protein:protein ratio of said polyclonal antibody to said monoclonal antibody of 5:1 to 8:1.

8. A support as claimed in Claim 7, wherein said ratio is about 7:1.

9. An immunoassay method in which a first member of a specific binding pair is detected by the reaction of said first member of said specific binding pair with the second member of said pair to form immune complexes, said second member being conjugated with an enzyme, characterized in that said conjugate of said second member and said enzyme is stabilized, prior to said reaction, by a stabilization buffer containing a chelated transition metal and being substantially free of sulphate and phosphate ions.

10. An immunoassay as claimed in Claim 9, wherein said second member is conjugated with said enzyme via a g-MBS-SATA linkage.

European Patent Office

# EUROPEAN SEARCH REPORT

Application number

| | DOCUMENTS CONSIDERED TO BE RELEVANT | | EP 86305828.5 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | EP - A2 - 0 123 902 (BYK-MALLINCK-RODT CHEMISCHE PRODUKTE GMBH) | 1 | G 01 N 33/52 |
| | * Page 13, lines 15-18 * | | G 01 N 33/535 |
| | | | G 01 N 33/536 |
| Y | * Page 14, example II; claim 1 * | 4 | G 01 N 33/577 |
| | -- | | C 07 C 87/50 |
| Y | GB - A - 1 563 299 (RAFA LABORA-TORIES LTD.) | 4 | |
| | * Example 3; claims 1-3 * | | |
| | ---- | | |

| | TECHNICAL FIELDS SEARCHED (Int. Cl.4) |
|---|---|
| | G 01 N 33/00 |
| | C 07 C 87/00 |

The present search report has been drawn up for all claims

| Place of search | Date of completion of the search | Examiner |
|---|---|---|
| VIENNA | 15-10-1986 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82